(19) Europäisches Patentamt
European Patent Office
Office européen des brevets

(11) EP 0 847 752 B1

(12) FASCICULE DE BREVET EUROPEEN

(45) Date de publication et mention de la délivrance du brevet:
**27.06.2001 Bulletin 2001/26**

(51) Int Cl.$^7$: **A61K 7/48**

(21) Numéro de dépôt: **97402768.2**

(22) Date de dépôt: **18.11.1997**

# (54) Composition topique sans transfert comprenant un composé fluorosiliconé

Abriebfeste topische Zusammensetzung enthaltend eine Fluorsilikon-Verbindung

Without rub off topical composition containing a fluorosilicon compound

(84) Etats contractants désignés:
**AT BE CH DE ES FR GB IT LI NL SE**

(30) Priorité: **26.11.1996 FR 9614484**

(43) Date de publication de la demande:
**17.06.1998 Bulletin 1998/25**

(60) Demande divisionnaire:
**01400303.2**

(73) Titulaire: **L'OREAL**
**75008 Paris (FR)**

(72) Inventeurs:
- **Arnaud, Pascal**
  **94000 Creteil (FR)**
- **Beaumard, Sophie**
  **94550 Chevilly Larue (FR)**

(74) Mandataire: **Lhoste, Catherine**
**L'OREAL-DPI**
**6 rue Bertrand Sincholle**
**92585 Clichy Cédex (FR)**

(56) Documents cités:
**EP-A- 0 590 192**
**EP-A- 0 602 905**
**EP-A- 0 620 270**
**EP-A- 0 657 486**
**EP-A- 0 661 042**
**WO-A-94/27568**
**WO-A-96/40044**
**WO-A-97/16157**
**FR-A- 2 689 010**
**JP-A- 8 109 121**
**US-A- 5 374 372**

- **STN, Serveur de Bases de Données, XP002040202 & JP 07 277 927 A (SHISEIDO CO)**
- **PATENT ABSTRACTS OF JAPAN vol. 018, no. 437 (C-1238) & JP 06 135818 A (SHIEIDO CO)**
- **Traduction du brevet JP8109121 A en français**
- **M. HANSEN: "The three dimensional solubility parameters - Key to paint component affinities", JOURNAL OF PAINT TECHNOLOGY, , Février 1967, Vol. 39, no. 505, pages 104 à 117**
- **D.W. VAN KREVELEN: "Propeties of polymers", 1990, ELSEVIER, AMSTERDAM-LONDON-NEW-YORK-TOKYO, 189-213**
- **R.F. FEDORS: "A method for estimating both the solubility parameters and molar volumes of liquids", POLYMER ENGINEERING AND SCIENCE, , Février 1974, Vol. 14, no. 2, pages 147 à 154**
- **C. WILLEMIN: "Paramètres de solubilité. Caractérisation et sélection des silicones", PARFUMS, COSMETIQUES ET ARôMES, , Décembre 1991, Vol. , no. 102, pages 57 à 63**
- **STN, Serveur de Bases de Données, XP002040202, Karlsruhe, DE, Fichier Chemical Abstracts, vol. 124, AN=97278 & JP 07 277 927 A (SHISEIDO CO)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 847 752 B1

## Description

La présente invention a pour objet une composition topique notamment cosmétique pouvant se présenter sous forme d'un stick ou d'une pâte souple, et susceptible d'être utilisée pour le soin et/ou le maquillage de la peau, des semi-muqueuses (comme les lèvres) et/ou des muqueuses (comme l'intérieur des paupières inférieures) des êtres humains, et plus spécialement des lèvres du visage.

Les compositions cosmétiques ou pharmaceutiques telles que les rouges à lèvres et les fonds de teint, comprennent généralement des corps gras tels que des huiles, des composés pâteux et des cires, ainsi qu'une phase particulaire généralement composée de charges et de pigments. Elles peuvent aussi contenir des actifs cosmétiques ou dermatologiques (vitamines, filtres, hydratants).

Ces compositions, lorsqu'elles sont appliquées sur la peau, les muqueuses ou les semi-muqueuses, présentent l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau ou les muqueuses, nécessitant de renouveler régulièrement son application. Par ailleurs, l'apparition de traces inacceptables sur certains vêtements et notamment sur les cols de chemisier peut écarter certaines femmes de l'utilisation de ce type de maquillage.

Un autre inconvénient de ces compositions réside dans le problème de migration. On a en effet constaté que certaines compositions avaient tendance à se propager à l'intérieur des ridules et/ou des rides de la peau, dans le cas des fonds de teint ; dans les ridules qui entourent les lèvres, dans le cas des rouges à lèvres ; dans les plis de la paupière, dans le cas des fards à paupières. On a également constaté, dans le cas notamment des fards à paupières, l'apparition de stries dans le maquillage, dues aux mouvements des paupières.

Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé, dans la demande de brevet JP-A-61-65809, des compositions de rouge à lèvres 'sans transfert' contenant de 1 à 70% en poids de résine pulvérulente de silicone à motifs répétitifs silicates (ou à réseau tridimensionnel) comportant des chaînes pendantes alkylées de 1 à 6 atomes de carbone ou phénylées, de 10 à 98% en poids d'une huile de silicone volatile cyclique et des charges pulvérulentes. Ces compositions présentent toutefois l'inconvénient d'être liquides et donc peu commodes à utiliser, ou tout au moins loin du concept classique d'un rouge à lèvres en bâton, limitant ainsi le nombre de femmes susceptibles d'utiliser ce type de rouge à lèvres. De plus, le film obtenu sur les lèvres après évaporation de l'huile de silicone présente l'inconvénient de devenir inconfortable au cours du temps (sensation de dessèchement et de tiraillement).

Plus récemment, il a été envisagé dans la demande de brevet EP-A-602905 des rouges à lèvres 'sans transfert' contenant une silicone volatile, cyclique ou linéaire, et une résine de silicone comportant une chaîne estérifiée pendante ayant au moins 12 atomes de carbone. Le film de rouge à lèvres présente notamment l'inconvénient de manquer de confort à l'application, en particulier d'être trop sec.

Plus récemment encore, il a été envisagé d'utiliser dans ces compositions sans transfert des résines de silicone modifiées par des groupements fluorés (voir le document EP-A-661 042). Ces résines se présentant encore sous forme pulvérulente, conduisent aussi à des compositions peu confortables, desséchantes et tiraillant les lèvres ou la peau sur lesquelles elles sont appliquées. De plus, ces résines sont difficiles à mettre en oeuvre. Ainsi il est préférable de les mélanger, préalablement à leur utilisation, avec une huile de silicone volatile, ce qui implique une étape de prémélange supplémentaire, d'où un surcoût notable lors d'une réalisation industrielle. D'autre part, la présence nécessaire d'une huile siliconée pour introduire ladite résine engendre une contrainte supplémentaire de formulation.

D'une manière générale, on sait maintenant que, si l'association d'huiles volatiles avec certains composés notamment siliconés permet d'obtenir un résultat 'sans transfert' satisfaisant, elle présente néanmoins l'inconvénient de conduire, après évaporation des volatils, à un film dont le confort n'est pas optimal, notamment parce qu'il n'est pas possible d'ajouter des huiles autres que des huiles siliconées dans ces compositions tout en conservant une qualité de 'sans transfert' correcte. En effet, les huiles hydrocarbonées, qui sont connues pour apporter notamment du confort à une composition cosmétique ou dermatologique, ont comme inconvénient d'augmenter le transfert d'une telle composition.

Or, à la suite d'études approfondies, la demanderesse a mis en évidence que, de manière inattendue et surprenante, il était possible de préparer une composition cosmétique dite 'sans transfert' permettant en particulier l'obtention d'un film ne transférant pas, ne migrant pas, et présentant des propriétés cosmétiques améliorées par rapport à celles des produits 'sans transfert' de l'art antérieur notamment des propriétés de glissant, de non tiraillement, de douceur et de confort.

Cette composition à confort amélioré par rapport à l'art antérieur permet de limiter le transfert et/ou la migration de ladite composition, et donc permet d'en améliorer la tenue mécanique, notamment au frottement et/ou à la pression.

Il est, en outre, connu du document EP-A-0 657 486 des compositions cosmétiques contenant des silicones fluorées ayant des propriétés de résistance à l'huile et à l'eau.

Un objet de la présente invention est donc une composition topique sans transfert anhydre de maquillage de la peau, des semi-muqueuses, des muqueuses et/ou des phanères telle que définie dans la revendication 1.

Un autre objet de l'invention est l'utilisation, dans une composition topique de maquillage de la peau, des semi-muqueuses, des muqueuses et/ou des phanères sans transfert, d'une phase grasse, telle que mentionnée ci-dessus, comme agent pour obtenir une composition présentant de bonnes propriétés de glissant, de non tiraillement, de douceur et/ou de confort.

L'invention a aussi pour objet un procédé cosmétique non thérapeutique tel que défini dans la revendication 22.

Par 'composition sans transfert', on entend notamment dans la présente description une composition qui ne transfère pas ou peu, c'est-à-dire qui ne se dépose pas et/ou ne tache pas et/ou n'adhère pas sur un support avec lequel elle est mise directement en contact. Notamment sont considérées comme 'non transfert' selon la présente invention, une composition de type fond de teint ou crème teintée qui ne tache pas un col de chemise (donc notamment résistante au frottement du au mouvement du visage et du cou), ainsi qu'une composition de type rouge à lèvres qui ne tache pas un support tel qu'un verre, une tasse ou de la peau (donc résistante à la pression due à l'application des lèvres sur ledit support).

L'invention a encore pour objet un procédé pour limiter, diminuer et/ou empêcher le transfert d'une composition de maquillage ou de soin de la peau, des muqueuses, des semi-muqueuses ou des phanères consistant à introduite dans la composition une phase grasse telle que définie ci-dessus.

La composition de la présente invention présente une tenue mécanique, notamment au frottement et/ou à la pression, donc une résistance au frottement, adéquate, et est jugée comme très confortable, à l'application et tout au long de la journée.

La composition selon l'invention trouve notamment une application particulièrement intéressante dans le domaine du soin et/ou du maquillage de la peau, des muqueuses, des semi-muqueuses et des phanères. On entend notamment par muqueuse, la partie interne de la paupière inférieure, parmi les semi-muqueuses, on entend plus particulièrement les lèvres du visage, par phanères, on entend les cils, sourcils, cheveux et ongles.

Ainsi, l'invention trouve une application toute particulière dans le domaine des produits de maquillage des lèvres du visage, mais aussi des produits de soin des lèvres ainsi que dans le domaine des produits de maquillage et de soin de la peau tels que les fonds de teint, les produits anti-cernes, les autobronzants ou les produits solaires.

Aussi, l'invention a encore pour objet une composition sans transfert de maquillage ou de soin des lèvres contenant une phase grasse telle que définie précédemment.

La composition selon l'invention comprend donc au moins un composé fluorosiliconé, qui peut être un composé volatil, un corps gras liquide non volatil, une cire, ou un mélange de ces composés. Dans un mode de réalisation préféré, le composé fluorosiliconé se présente sous forme d'un corps gras liquide non volatil.

L'association particulière du composé fluorosiliconé avec des huiles et/ou cires particulières, le tout associé à un composé volatil permet d'obtenir des propriétés remarquables de 'sans transfert' tout en conduisant à un film d'un très grand confort sur le support sur lequel il est appliqué.

Lors de la préparation d'une composition selon l'invention, on obtient un mélange homogène dans lequel le composé fluorosiliconé est parfaitement solubilisé ou dispersé. Lorsque la composition est appliquée sur un support, par exemple sur les lèvres, les composés volatils s'évaporent rapidement. Sans être tenu par la présente explication on peut envisager que, dans la composition restant sur les lèvres, le composé fluorosiliconé aura tendance à migrer vers la partie la plus éloignée dudit support, autrement dit aura tendance à 'remonter' à la surface du film déposé, ce qui aura pour conséquence de laisser dans la partie la plus proche du support, et donc en contact direct avec ledit support, principalement les autres composés gras et notamment les huiles hydrocarbonées qui apportent le confort.

Le composé fluorosiliconé utilisé dans la composition selon l'invention a la formule suivante (III) :

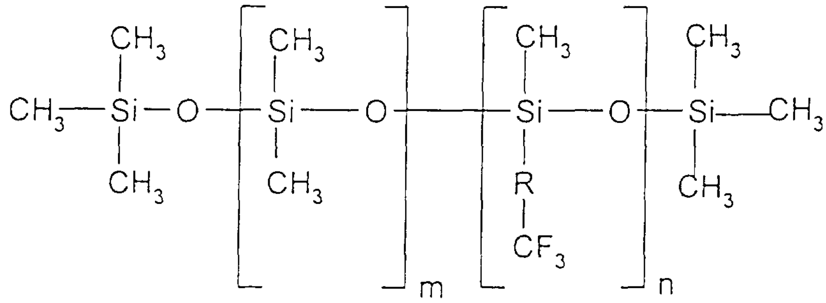

avec

- R représentant un groupement divalent méthyle, éthyle, propyle ou butyle
- m étant choisi de 0 à 80, et
- n étant choisi de 1 à 30.

De tels composés sont notamment ceux commercialisés par Shin Etsu sous les dénominations 'X22-819', 'X22-820', 'X22-821' et'X22-822' ou encore 'FL-100'.

On peut bien évidemment utiliser un mélange de plusieurs composés fluorosiliconés. Les composés huileux non volatils peuvent notamment remplacer tout ou partie des corps gras liquides de la composition.

La composition selon l'invention peut comprendre par ailleurs tout composé connu de l'homme du métier pour le type d'application envisagé, ne détruisant pas les propriétés recherchées.

Dans un mode de réalisation préféré, la composition est anhydre.

Le composé fluorosiliconé peut être présent dans la composition à raison de 0,1 à 40% en poids, de préférence 3 à 30% en poids par rapport au poids total de la composition.

La composition selon l'invention comprend au moins un composé volatil à température ambiante (20-25°C), qui peut donc être fluoré ou non. Par composé volatil, on entend dans la présente description, tout composé susceptible de s'évaporer au contact de la peau. De préférence, on utilise des huiles volatiles dont le point éclair est suffisamment élevé pour permettre l'utilisation de ces huiles en formulation, et suffisamment bas pour obtenir l'effet évanescent souhaité. On emploie de préférence des huiles volatiles dont le point éclair est de l'ordre de 40-100°C.

Ces composés volatils peuvent être choisis en particulier parmi les huiles hydrocarbonées et/ou les huiles siliconées, cycliques ou linéaires, seules ou en mélange.

Parmi les huiles siliconées volatiles, on peut citer, seules ou en mélange,

- les silicones volatiles cycliques ayant de 3 à 8 atomes de silicium et de préférence de 4 à 6. Il s'agit par exemple de la cyclotétradiméthylsiloxane, de la cyclopentadiméthylsiloxane ou de la cyclohexadiméthylsiloxane
- les cyclocopolymères du type diméthylsiloxane/méthylalkylsiloxane, tels que la SILICONE FZ 3109 vendue par la société UNION CARBIDE, qui est un cyclocopolymère diméthylsiloxane/méthyloctylsiloxane,
- les silicones volatiles linéaires ayant de 2 à 9 atomes de silicium. Il s'agit par exemple de l'hexaméthyldisiloxane ou un PDMS de faible viscosité (1 cSt). On peut encore citer les alkyltrisiloxanes tels que l'hexylheptaméthyltrisiloxane ou l'octylheptaméthyltrisiloxane.

Parmi les huiles hydrocarbonées volatiles, on peut citer les isoparaffines en $C_8$-$C_{16}$ et notamment l'isododécane.

La composition selon l'invention peut comprendre 8 à 99% en poids, de préférence 15 à 85% en poids et plus préférentiellement 30 à 70% en poids, de composés volatils, par rapport au poids total de la composition.

La composition comprend également au moins une cire qui peut être fluorée ou non, ou un mélange de différentes cires, de manière à aider à assurer notamment la résistance mécanique de la composition, lorsqu'elle se présente sous la forme d'un stick. Lorsqu'elle se présente sous la forme d'une pâte souple ou d'un produit coulé, la composition selon l'invention peut comprendre une quantité moins importante de cire.

On peut employer toute cire connue dans l'art antérieur parmi lesquelles, seules ou en mélange, on peut citer les cires d'origine animale, végétale, minérale et synthétique, telles que les cires microcristallines, la paraffine, le pétrolatum, la vaseline, l'ozokérite, la cire de montan, la cire d'abeilles, la lanoline et ses dérivés, les cires de Candellila, d'Ouricury, de Carnauba, du Japon, le beurre de cacao, les cires de fibres de lièges ou de canne à sucre, les huiles hydrogénées concrètes à 25°C, les ozokérites, les esters gras et les glycérides concrets à 25°C, les cires de polyéthylène et les cires obtenues par synthèse de Fischer-Tropsch, les cires de silicone et notamment les cires alkylsiliconées, ainsi que les cires fluorées, leurs mélanges.

De préférence, les cires entrant dans la composition peuvent présenter un point de fusion supérieur à 45°C environ, et en particulier supérieur à 55°C, et/ou un indice de pénétration de l'aiguille à 25°C de préférence compris entre 3 et 40.

De préférence, la composition comprend 0,5 à 30% en poids de cire, notamment 5 à 20% en poids, par rapport au poids total de la composition.

La composition comprend par ailleurs au moins un corps gras liquide non volatil, qui peut être fluoré ou non. Par corps gras liquide, on entend un composé ayant un point de fusion inférieur à environ 30-35°C, par opposition aux corps gras solides, tels que les cires, qui ont un point de fusion supérieur à environ 50°C.

Parmi les corps gras liquides envisageables, on peut citer tout corps gras liquide non volatil connu de l'homme du métier pour l'application envisagée. On peut notamment citer les huiles d'origine végétale, minérale, animale, synthétique et/ou siliconée, leurs mélanges.

Parmi les huiles siliconées, on peut citer les huiles de silicone phénylées, notamment de type polyphénylméthylsiloxane ou phényltriméthicone, et en particulier les huiles répondant à la formule suivante

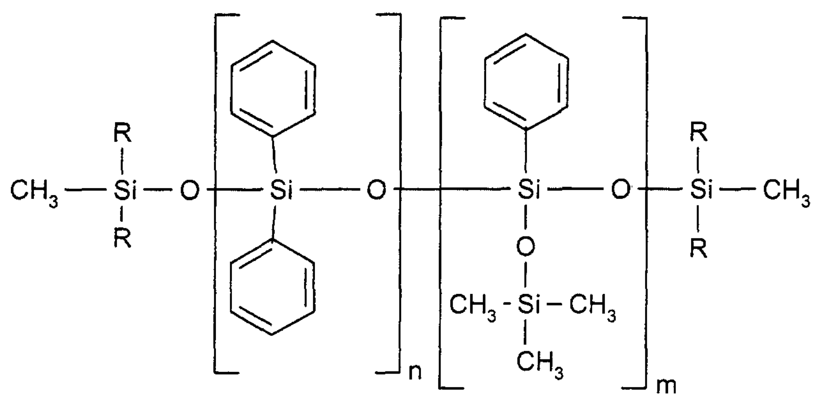

(i)

dans laquelle

- R est un radical alkyle en C1-C30 linéaire, ramifié, saturé ou non, un radical aryle ou un radical aralkyle, en C7 et C60
- n est un nombre entier allant de 0 à 100,
- m est un nombre entier allant de 0 à 100, sous réserve que la somme m+n est choisi de 1 à 100.

On peut encore citer les polyalkyl($C_1$-$C_{20}$) siloxanes dans lequel le groupement alkyle est linéaire, ramifié, saturé ou non, et notamment ceux à groupements terminaux triméthylsilyle, parmi lesquels on peut citer les polydiméthylsiloxanes linéaires et les alkylméthylpolysiloxanes, les alkyldiméthicones, les silicones modifiées par des groupements aliphatiques et/ou aromatiques, ou par des groupements fonctionnels tels que des groupements hydroxyles, thiols et/ou amines.

Parmi les huiles hydrocarbonées d'origine animale, végétale, minérale ou de synthèse, on peut citer les huiles formées par des esters d'acide gras et de polyols, en particulier les triglycérides liquides, par exemple les huiles de tournesol, de maïs, de soja, de courge, l'huile de paraffine, de vaseline, le perhydrosqualène, l'huile d'arachide, d'amande douce, de macadamia, de pépuis de raisin, de colza, de coprah, de calophyllum, de palme, de ricin, d'avocat, d'abricot, de sésame, de jojoba, d'olive ou de germes de céréales, les huiles de poisson, le tricaprocaprylate de glycérol, des esters d'acides gras, des alcools, des acétylglycérides, des octanoates, décanoates ou ricinoléates d'alcools ou de polyalcools, des triglycérides d'acides gras, des glycérides, les huiles de formule $R_1COOR_2$ dans laquelle $R_1$ représente le reste d'un acide gras supérieur comportant de 7 à 19 atomes de carbone et $R_2$ représente une chaîne hydrocarbonée ramifiée contenant de 3 à 20 atomes de carbone, par exemple l'huile de Purcellin ou l'huile de germes de blé, leurs mélanges.

Dans un mode de réalisation préféré, les huiles présentes dans la composition sont majoritairement hydrocarbonées.

De préférence, la composition selon l'invention peut comprendre 1 à 50% en poids de corps gras liquide non volatil, notamment de 1 à 40 % et mieux de 5 à 30% en poids.

Parmi les autres corps gras susceptibles d'être présents dans la composition, on peut citer les gommes de

silicone ainsi que les corps gras pâteux d'origine végétale, minérale, animale, synthétique et/ou siliconée. Ces corps gras peuvent en particulier être choisis de manière variée par l'homme du métier afin de préparer une composition ayant les propriétés souhaitées, par exemple en consistance ou en texture. Ces corps gras peuvent être aussi bien hydrocarbonés que siliconés, ce qui permet d'adapter les propriétés du film notamment en ce qui concerne le confort sur les lèvres ou la peau des êtres humains.

Le(s) corps gras liquide(s) non volatil(s) non fluoré(s) et/ou la/les cire(s) non fluorée(s) sont choisis de manière à vérifier la relation suivante :

$$0 \leq \Delta\delta \leq 5$$

et de préférence

$$\Delta\delta \leq 4$$

La distance $\Delta\delta$ représente la distance dans l'espace de Hansen, entre le point représentatif de la cire, ou du mélange de cires, et le point représentatif du corps gras liquide non volatil, ou du mélange de corps gras liquides non volatils.

Les composés volatils, les corps gras liquides non volatils fluorés et les cires fluorées ne sont pas prises en compte lors du calcul de la distance $\Delta\delta$.

La distance $\Delta\delta$ peut être calculée de la manière suivante :

$$\Delta\delta = [4 \times (\delta D \text{ cires} - \delta D \text{ huiles})^2 + (\delta P \text{ cires} - \delta P \text{ huiles})^2 + (\delta H \text{ cires} - \delta H \text{ huiles})^2]^{1/2}$$

La définition des corps gras dans l'espace de solubilité tridimensionnel selon HANSEN est décrite dans l'article de C. M. HANSEN : "The three dimensional solubility parameters" J. Paint Technol. 39, 105 (1967).

Selon cet espace de Hansen :

- $\delta D$ caractérise les forces de dispersion de LONDON issues de la formation de dipôles induits lors des chocs moléculaires ;
- $\delta P$ caractérise les forces d'interactions de DEBYE entre dipôles permanents ;
- $\delta H$ caractérise les forces d'interactions spécifiques (type liaisons hydrogène, acide/base, donneur/accepteur, etc.) ;

Les paramètres $\delta P$, $\delta H$ et $\delta D$ sont généralement exprimés en $(J/cm^3)^{1/2}$.

Dans la composition selon l'invention, on peut utiliser n'importe quel corps gras ou mélange de corps satisfaisant aux relations ci-dessus. Dans ce cas, les paramètres de solubilité du mélange sont déterminés à partir de ceux des corps gras pris séparément, selon les relations suivantes :

$$\delta_{Dmel} = \sum_i xi\, \delta_{Di} \quad ; \quad \delta_{pmel} = \sum_i xi\, \delta_{pi} \quad \text{et} \quad \delta_{hmel} = \sum_i xi\, \delta_{hi}$$

où xi représente la fraction volumique du corps gras i dans le mélange.

Il est à la portée de l'homme du métier de déterminer les quantités de chaque corps gras pour obtenir un mélange de corps gras satisfaisant aux relations ci-dessus.

La composition comprend également une phase particulaire, qui peut comprendre des pigments et/ou des nacres et/ou des charges habituellement utilisés dans les compositions cosmétiques et dermatologiques. Par pigments, il faut comprendre des particules blanches ou colorées, minérales ou organiques, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition, et/ou de la douceur, de la matité et de l'uniformité au maquillage. Par nacres, il faut comprendre des particules irisées qui réfléchissent la lumière.

Les pigments peuvent être présents dans la composition à raison de 0 à 15% en poids de la composition finale, et de préférence à raison de 3 à 12% en poids. Ils peuvent être blancs ou colorés, minéraux et/ou organiques,

de taille usuelle ou nanométrique. On peut citer les dioxydes de titane, de zirconium ou de cérium, ainsi que les oxydes de zinc, de fer ou de chrome, le bleu ferrique, le noir de carbone, les outremers (polysulfures d'aluminosilicates), le pyrophosphate de manganèse et certaines poudres métalliques telles que celles d'argent ou d'aluminium. On peut encore citer les laques couramment employées pour conférer aux lèvres et à la peau un effet de maquillage, qui sont des sels de calcium, de baryum, d'aluminium ou de zirconium, de colorants acides tels que les colorants halogénoacides, azoïques, anthraquinoniques, etc.

Les nacres peuvent être présentes dans la composition à raison de 0 à 20% en poids, de préférence à un taux de l'ordre de 1 à 10% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

Les charges, qui peuvent être présentes à raison de 0 à 30% en poids, de préférence 1 à 15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le nitrure de bore, les microsphères telles que l'Expancel (Nobel Industrie), le polytrap (Dow Corning) et les microbilles de résine de silicone (Tospearls de Toshiba, par exemple).

La composition peut comprendre en outre tout additif usuellement utilisé dans le domaine cosmétique ou dermatologique, tel que des antioxydants, des parfums, des huiles essentielles, des conservateurs, des épaississants, des actifs cosmétiques, des hydratants, des vitamines, des colorants, des acides gras essentiels, des sphingolipides, des agents autobronzants tels que la DHA, des filtres solaires.

La composition selon l'invention peut également comprendre au moins un agent actif, parmi lesquels on peut citer les agents actifs contre les micro-organismes, notamment à activité antivirale, antibactérienne ou antifongique, les agents à activité anti-inflammatoire ou immunomodulatrice, les agents antagonistes des neuromédiateurs ou modulant le relarguage des neuromédiateurs, les agents modulant la différenciation et/ou la prolifération cellulaire et/ou la pigmentation et/ou régulant la kératinisation, les agents actifs dans le traitement et/ou la prévention des cheilites, les antihistaminiques, les agents cicatrisants.

Bien entendu l'homme du métier veillera à choisir les éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter sous la forme d'un stick ou bâton, sous la forme d'un liquide huileux, éventuellement gélifié, ou encore sous la forme d'une pâte souple dont on peut mesurer la viscosité, ladite viscosité dynamique à 25°C étant généralement comprise entre 1 et 40 Pa.s, mesurée avec un viscosimètre rotatif CONTRAVES TV équipé d'un mobile "MS-r4" à la fréquence de 60 Hz et mieux allant de 3 à 35 Pa.s.

Les compositions selon l'invention trouvent une application notamment dans le domaine du maquillage de la peau, des semi-muqueuses, des muqueuses et/ou des phanères, et se présentent alors par exemple sous la forme d'un rouge à lèvres, d'un fond de teint, d'un fard à joues ou à paupières, d'un mascara ou d'un eye-liner, d'un produit anti-cerne.

Elles peuvent également se présenter sous forme non colorée, contenant éventuellement des actifs cosmétiques ou dermatologiques. Elles peuvent alors notamment être utilisées comme base de soin pour les lèvres ou comme base fixante d'un film de rouge à lèvres, pour limiter le transfert et la migration d'un film de soin ou de maquillage, et d'augmenter ainsi sa tenue.

Les compositions selon l'invention peuvent également se présenter sous la forme d'un produit de soin de la peau, des muqueuses, des semi-muqueuses et/ou des phanères, tel qu'un gel, une crème, un baume ou une lotion, d'un produit hygiénique ou pharmaceutique ou encore d'un produit solaire ou autobronzant.

L'invention est illustrée plus en détail dans les exemples suivants.

<u>Exemple 1</u>

On prépare un bâton de rouge à lèvres ayant la composition suivante

. silicone fluorée (X22819 de Shin Etsu) 8 g
. cire de polyéthylène 16 g
. polyisobutène hydrogéné 12 g
. propionate d'arachidyle 9,5 g
. pigments 9,5 g
. cyclotétrapolysiloxane qsp 100 g

On prépare la composition de manière usuelle, en chauffant la cire, le polyisobutène et le propionate d'arachidyle à 95°C et en les mélangeant. On ajoute ensuite la silicone fluorée et les pigments puis, à 60°C, l'huile volatile siliconée. On mélange le tout à l'aide d'une turbine Moritz à la vitesse de 3000 tr/min. On peut alors couler le mélange

homogène obtenu à 85°C dans des moules adéquats.

On obtient après refroidissement, un bâton de rouge à lèvres de texture agréable, qui s'applique uniformément sur les lèvres et dont le film est très confortable, tout en ne laissant pas de traces sur des supports extérieurs après évaporation de l'huile volatile (quelques minutes). Ceci est d'autant plus remarquable que la composition comprend une quantité importante d'huile hydrocarbonée, peu favorable au 'sans transfert'.

On applique cette composition sur la partie gauche des lèvres de plusieurs personnes. Pour comparaison, on applique sur la partie droite desdites lèvres, un rouge à lèvres 'sans transfert' de l'art antérieur (Colour Endure de l'Oréal). On laisse sécher les rouges à lèvres à température ambiante pendant 10 minutes, puis on applique la totalité des lèvres sur une feuille de papier.

On constate sur la totalité des feuilles de papier une trace de rouge à lèvres très faible, à peine perceptible, aussi bien pour la composition de l'invention que pour la composition de l'art antérieur.

La valeur $\Delta\delta$ (cire de polyéthylène et polyisobutène hydrogéné) est de 1,62.

<u>Exemple 2</u>

On prépare comme dans l'exemple 1, un bâton de rouge à lèvres ayant la composition suivante

. silicone fluorée (X22820 de Shin Etsu) 8 g
. cire de polyéthylène 16 9
. squalane 12 g
. propionate d'arachidyle 9,5 g
. pigments 9,5 g
. cyclotétrapolysiloxane qsp 100 g

On obtient un bâton de rouge à lèvres permettant l'obtention d'un film confortable et présentant à la fois des propriétés de 'sans transfert' remarquables.

La valeur $\Delta\delta$ (cire de polyéthylène et squalane) est de 1,17.

<u>Exemple 3</u>

On prépare selon l'exemple 1, un bâton de rouge à lèvres ayant la composition suivante

. silicone fluorée (X22819 de Shin Etsu) 16 g
. cire de polyéthylène 16 g
. polyisobutène hydrogéné 4 g
. propionate d'arachidyle 9,5 g
. pigments 9,5 g
. cyclotétrapolysiloxane qsp 100 g

La valeur $\Delta\delta$ (cire de polyéthylène et polyisobutène hydrogéné) est de 1,62.

On obtient un bâton de rouge à lèvres permettant l'obtention d'un film confortable et présentant à la fois des propriétés de 'sans transfert' remarquables.

## Revendications

**1.** Composition topique sans transfert de maquillage de la peau, des semi-muqueuses, des muqueuses et/ou des phanères sous forme anhydre comprenant une phase grasse comprenant de 8 à 99 % en poids de composé volatil, au moins une cire, au moins un corps gras liquide non volatil, et une phase particulaire:

- la phase grasse comprenant au moins un composé fluorosiliconé de formule (III):

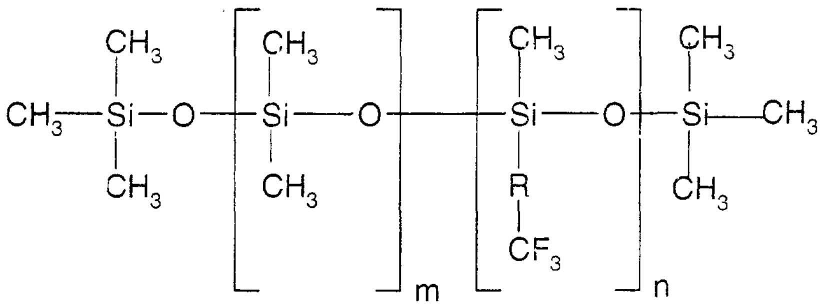

avec

- R représentant un groupement divalent méthyle, éthyle, propyle ou butyle
- m étant choisi de 0 à 80, et
- n étant compris de 1 à 30, et
- le(s) corps gras liquide(s) non volatil(s), et la/les cire(s) sont choisis de manière à vérifier la relation suivante :

$$0 \leq \Delta\delta \leq 5$$

dans laquelle :

$$\Delta\delta = [4x(\delta D \text{ cires} - \delta D \text{ huiles})^2 + (\delta P \text{ cires} - \delta P \text{ huiles})^2 + (\delta H \text{ cires} - \delta H \text{ huiles})^2]^{1/2}, \text{ où :}$$

- δD caractérise les forces de dispersion de LONDON issues de la formation de dipôles induits lors des chocs moléculaires ;
- δP caractérise les forces d'interactions de DEBYE entre dipôles permanents ;
- δH caractérise les forces d'interactions spécifiques ; et
- la phase particulaire contenant de 0 à 15 % en poids de pigments, de 0 à 20 % en poids de nacres et de 0 à 30 % en poids de charges, par rapport au poids total de la composition.

**2.** Composition selon la revendication 1, dans laquelle le composé fluorosiliconé est choisi parmi les composés volatils, les corps gras liquides non volatils, les cires, et leurs mélanges.

**3.** Composition selon l'une des revendications précédentes, dans laquelle le composé fluorosiliconé se présente sous forme d'un corps gras liquide non volatil.

**4.** Composition selon l'une des revendications précédentes, dans laquelle la phase contient au moins une cire hydrocarbonée fluorée.

**5.** Composition selon l'une des revendications précédentes, dans laquelle le composé fluorosiliconé est présent dans la composition à raison de 0,1 à 40% en poids, de préférence 3 à 30% en poids.

**6.** Composition selon l'une des revendications précédentes, dans laquelle le composé volatil est choisi parmi les huiles hydrocarbonées et/ou les huiles siliconées, cycliques ou linéaires, seules ou en mélange.

**7.** Composition selon l'une des revendications précédentes, dans laquelle la cire est choisie parmi, seule ou en mélange, les cires d'origine animale, végétale, minérale et synthétique, telles que les cires microcristallines, la paraffine, le pétrolatum, la vaseline, l'ozokérite, la cire de montan, la cire d'abeilles, la lanoline et ses dérivés, les cires de Candellila, d'Ouricury, de Carnauba, du Japon. le beurre de cacao, les cires de fibres de lièges ou de canne à sucre, les huiles hydrogénées concrètes à 25°C, les ozokérites, les esters gras et les glycérides concrets à 25°C, les cires de polyéthylène et les cires obtenues par synthèse de Fischer-Tropsch, les cires de silicone et notamment les cires alkylsiliconées.

**8.** Composition selon l'une des revendications précédentes, comprenant en outre au moins une huile choisie parmi

les huiles non volatiles hydrocarbonées et/ou les huiles non volatiles siliconées phénylées.

**9.** Composition selon l'une des revendications précédentes, dans laquelle les huiles non volatiles présentes dans la composition sont majoritairement hydrocarbonées.

**10.** Composition selon l'une des revendications précédentes, dans laquelle $\Delta\delta \leq 4$.

**11.** Composition selon l'une des revendications précédentes, se présentant sous la forme d'un stick ou bâton, sous la forme d'un liquide huileux, éventuellement gélifié, sous la forme d'une pâte souple de viscosité dynamique à 25°C allant de 1 à 40 Pa.s et mieux de 3 à 35 Pa.s, ladite viscosité étant mesurée avec un viscosimètre rotatif CONTRAVES TV équipé d'un mobile "MS-r4" à la fréquence de 60 Hz.

**12.** Composition selon l'une des revendications précédentes, se présentant sous la forme d'un rouge à lèvres, d'un fond de teint, d'un fard à loues ou à paupières, d'un mascara, d'un eye-liner, d'une base de soin pour les lèvres, d'une base fixant, d'un produit de soin, d'un produit hygiénique ou pharmaceutique, d'un produit solaire ou autobronzant.

**13.** Composition selon l'une des revendications précédentes, caractérisée en ce qu'elle est une composition pour le maquillage des lèvres.

**14.** Utilisation, dans une composition topique sans transfert de maquillage de la peau, des semi-muqueuses, des muqueuses et/ou des phanères et sous forme anhydre, d'une phase grasse comprenant au moins un composé fluorosiliconé, de 8 à 99% en poids de composé volatil, au moins une cire, au moins un corps gras liquide non volatil et une phase particulaire, ledit/lesdits corps gras liquide(s) non volatil(s) et ladite/lesdites cire(s) étant choisis de manière à vérifier la relation suivante : $0 \leq \Delta\delta \leq 5$, $\Delta\delta$ ayant la définition mentionnée dans la revendication 1, le composé fluorosiliconé étant de formule (111)

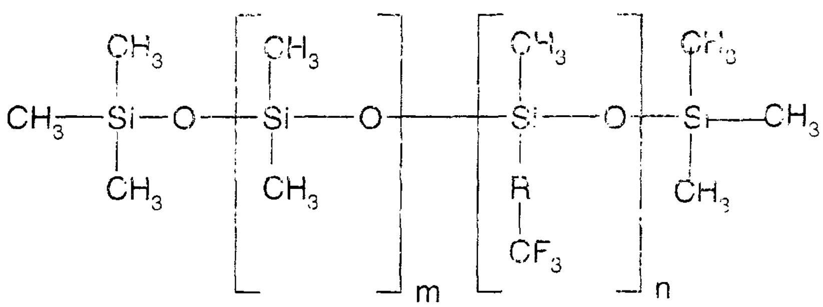

avec

- R représentant un groupernent divalent méthyle, éthyle, propyle ou butyle
- m étarit choisi de 0 à 80, et
- n étant choisi de 1 à 30.

et la phase particulaire contenant de 0 à 15 % en polds de pigments, de 0 à 20 % en poids de nacres et de 0 à 30 % en poids de charges, par rapport au poids total de la composition, comme agent pour obtenir une composition présentant de bonnes propriétés de glissant, de non tiraillement, de douceur et/ou de confort.

**15.** Utilisation selon la revendication 14, dans laquelle le composé fluorosiliconé est choisi parmi les composés volatils, les corps gras liquides non volatils, les cires, et leurs mélanges.

**16.** Utilisation selon l'une des revendications 14 à 15, dans laquelle le composé comprend au moins une cire hydrocarbonée fluorée.

**17.** Utilisation selon l'une des revendications 14 à 16, dans laquelle le composé fluorosiliconé est présent dans la composition à raison de 0, 1 à 40% en poids, de préférence 3 à 30% en poids,

**18.** Utilisation selon l'une des revendications 14 à 17, dans laquelle la composition comprend en outre au moins une huile choisie parmi les huiles non volatiles hydrocarbonées et/ou les huiles non volatiles siliconées phénylées.

**19.** Utilisation selon l'une des revendication 14 à 18 dans laquelle les huiles non volatiles présentes dans la composition sont majoritairement hydrocarbonées.

**20.** Utilisation selon l'une des revendications 14 à 19, dans laquelle $\Delta\delta \leq 4$.

**21.** Utilisation selon l'une des revendications 14 à 20. dans laquelle la composition se présente sous la forme d'un rouge à lèvres, d'un fond de teint, d'un fard à joues ou à paupières, d'un mascara, d'un eye-liner, d'une base de soin pour les lèvres, d'une base fixante, d'un produit de soin, d'un produit hygiénique ou pharmaceutique, d'un produit solaire ou autobronzant.

**22.** Procédé cosmétique non thérapeutique pour limiter, diminuer et/ou empêcher le transfert d'une composition cosmétique anhydre de maquillage de la peau, des muqueuses, des semi-muqueuses ou des phanères, consistant à introduire dans la composition une phase grasse comprenant de 8 à 99% en polds de composé volatil, au moins une cire, au moins un corps gras liquide non volatil, et au moins un composé fluorosiliconé de formule (III) :

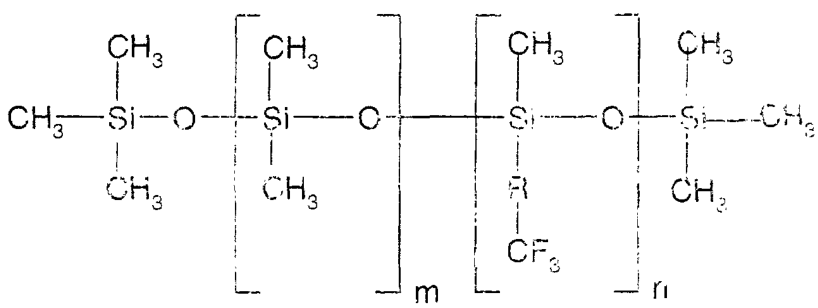

avec

- R représentant un groupement divalent méthyle, éthyle, propyle ou butyle
- m étant choisi de 0 à 80, et
- n étant choisi de 1 à 30, et
- le ou les corps gras liquides non volatils et la/les cire(s) sont choisis de manière à vérifier la relation suivante :

$$0 \leq \Delta\delta \leq 5$$

dans laquelle :

$$\Delta\delta = [4x(\delta D \text{ cires} - \delta D \text{ huiles})^2 + (\delta P \text{ cires} - \delta P \text{ huiles})^2 + (\delta H \text{ cires} - \delta H \text{ huiles})^2]^{1/2},$$

où :

- $\delta D$ caractérise les forces de dispersion de LONDON issues de la formation de dipôles induits lors des chocs moléculaires ;
- $\delta P$ caractérise les forces d'interactions de DEBYE entre dipôles permanents :
- $\delta H$ caractérise les forces d'interactions spécifiques ;

ladite composition comprenant une phase particulaire contenant de 0 à 15 % en poids de pigments, de 0 à 20 % en poids de nacres et de 0 à 30 % en poids de charges, par rapport au poids total de la composition.

## Claims

**1.** Transfer-free topical composition for making up the skin, semi-mucous membranes, mucous membranes and/or superficial body growths in the anhydrous form comprising a fatty phase comprising from 8 to 99% by weight of volatile compound, at least one wax, at least one non-volatile liquid fatty substance and a particulate phase:

- the fatty phase comprising at least one fluorosilicone compound of formula (III):

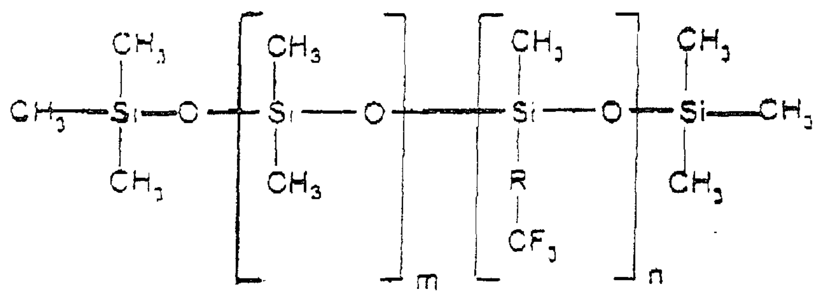

with

- R representing a divalent methylene, ethylene, propylene or butylene group
- m being chosen from 0 to 80, and
- n being chosen from 1 to 30, and
- the non-volatile liquid fatty substance(s) and the wax(es) being chosen so as to verify the following relationship:

$$0 \leq \delta \leq 5$$

in which:

$$\Delta\delta = (4 \times (\delta D \text{ waxes} - \delta D \text{ oils})^2 + (\delta P \text{ waxes} - \delta P \text{ oils})^2 - (\delta H \text{ waxes} - \delta H \text{ oils})^2]^4,$$

where:

- $\delta D$ characterizes the London dispersion forces resulting from the formation of dipoles induced during molecular impacts;
- $\delta P$ characterizes the Debye interaction forces between permanent dipoles;
- $\delta H$ characterizes the specific interaction forces; and
- the particulate phase comprising from 0 to 15% by weight of pigments, from 0 to 20% by weight of pearlescent agents and from 0 to 30% by weight of fillers, with respect to the total weight of the composition.

**2.** Composition according to Claim 1, in which the fluorosilicone compound is chosen from volatile compounds, non-volatile liquid fatty substances, waxes and their mixtures.

**3.** Composition according to one of the preceding claims, in which the fluorosilicone compound is provided in the form of a non-volatile liquid fatty substance.

**4.** Composition according to one of the preceding claims, in which the phase comprises at least one fluorinated hydrocarbonaceous wax.

**5.** Composition according to one of the preceding claims, in which the fluorosilicone compound is present in the composition in the proportion of 0.1 to 40% by weight, preferably 3 to 30% by weight.

**6.** Composition according to one of the preceding claims, in which the volatile compound is chosen, alone or as a mixture, from hydrocarbonaceous oils and/or silicone oils, which are cyclic or linear,

7. Composition according to one of the preceding claims, in which the wax is chosen, alone or as a mixture, from waxes of animal, vegetable, mineral and synthetic origin, such as microcrystalline waxes, paraffin wax, petrolatum wax, petroleum jelly, ozokerite, montan wax, beeswax, lanolin and its derivatives, candelilla, ouricury, carnauba and Japan waxes, cocoa butter, sugarcane or cork fibre waxes, hydrogenated oils which are solid at 25°C, ozokerites, fatty esters and glycerides which are solid at 25°C, polyethylene waxes and waxes obtained by the Fischer-Tropsch synthesis, silicone waxes and in particular alkylsilicone waxes.

8. Composition according to one of the preceding claims, additionally comprising at least one oil chosen from non-volatile hydrocarbonaceous oils and/or non-volatile phenylated silicone oils,

9. Composition according to one of the preceding claims, in which the non-volatile oils present in the composition are mainly hydrocarbonaceous oils.

10. Composition according to one of the preceding claims, in which $\Delta\delta \leq 4$.

11. Composition according to one of the preceding claims, which is provided in the form of a stick, in the form of an oily liquid, optionally gelled, or in the form of a supple paste with a dynamic viscosity at 25°C ranging from 1 to 40 Pa·s and better still from 3 to 35 Pa·s, the said viscosity being measured with a Contraves TV rotary viscometer equipped with an "MS-r4" rotor at a frequency of 60 Hz.

12. Composition according to one of the preceding claims, which is provided in the form of a lipstick, of a foundation, of a blusher, of an eyeshadow, of a mascara, of an eyeliner, of a care base for the lips, of a fixing base, of a care product, of a hygiene or pharmaceutical product or of an antisun or self-tanning product.

13. Composition according to one of the preceding claims, characterized in that it is a composition for making up the lips.

14. Use, in a transfer-free topical composition for making up the skin, semi-mucous membranes, mucous membranes and/or superficial body growths in the anhydrous form, of a fatty phase comprising at least one fluorosilicone compound, from 8 to 99% by weight of volatile compound, at least one wax, at least one nonvolatile liquid fatty substance and a particulate phase, the said non-volatile liquid fatty substance(s) and the said wax(es) being chosen so as to verify the following relationship: $0 \leq \Delta\delta \leq 5$, $\Delta\delta$ having the definition mentioned in Claim 1, the fluorosilicone compound being of formula (III) :

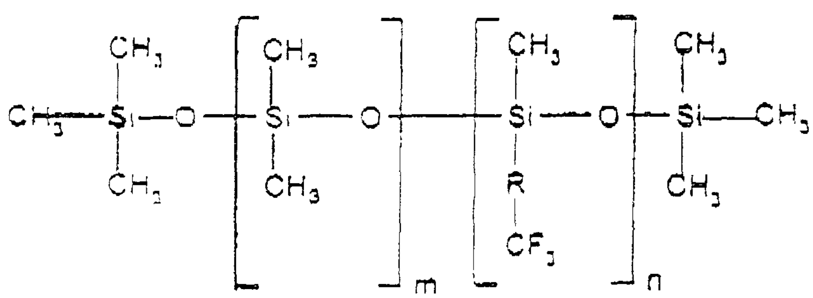

with

- R representing a divalent methylene, ethylene, propylene or butylene group
- m being chosen from 0 to 80, and
- n being chosen from 1 to 30,

and the particulate phase comprising from 0 to 15% by weight of pigments, from 0 to 20% by weight of pearlescent agents and from 0 to 30% by weight of fillers, with respect to the total weight of the composition, as agent for obtaining a composition exhibiting good slip, non-tightness, softness and/or comfort properties,

15. Use according to Claim 14, in which the fluorosilicone compound is chosen from volatile compounds, non-volatile liquid fatty substances, waxes and their mixtures.

16. Use according to either of Claims 14 and 15, in which the compound comprises at least one fluorinated hydrocarbonaceous wax.

**17.** Use according to one of Claims 14 to 16, in which the fluorosilicone compound is present in the composition in the proportion of 0.1 to 40% by weight, preferably 3 to 30% by weight.

**18.** Use according to one of Claims 14 to 17, in which the composition additionally comprises at least one oil chosen from non-volatile hydrocarbonaceous oils and/or non-volatile phenylated silicone oils.

**19.** Use according to one of Claims 14 to 18, in which the non-volatile oils present in the composition are mainly hydrocarbonaceous oils.

**20.** Use according to one of Claims 14 to 19, in which $\Delta\delta \leq 4$.

**21.** Use according to one of Claims 14 to 20, in which the composition is provided in the form of a lipstick, of a foundation, of a blusher, of an eyeshadow, of a mascara, of an eyeliner, of a care base for the lips, of a fixing base, of a care product, of a hygiene or pharmaceutical product or of an antisun or self-tanning product.

**22.** Non-therapeutic cosmetic process for limiting, decreasing and/or preventing the transfer of an anhydrous cosmetic composition for making up the skin, mucous membranes, semi-mucous membranes or superficial body growths which consists in introducing, into the composition, a fatty phase comprising from 8 to 99% by weight of volatile compound, at least one wax, at least one non-volatile liquid fatty substance and at least one fluorosilicone compound of formula (III):

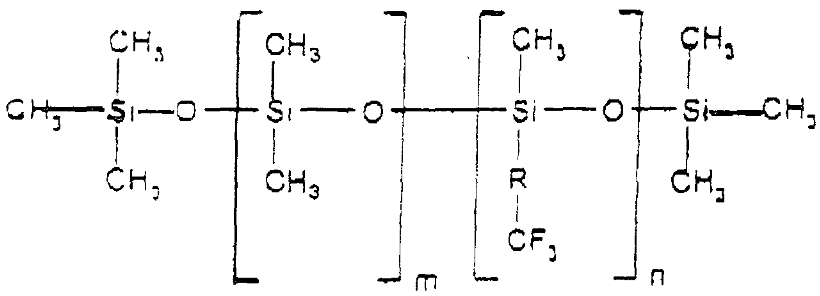

with

- R representing a divalent methylene, ethylene, propylene or butylene group
- m being chosen from 0 to 80, and
- n being chosen from 1 to 30, and
- the non-volatile liquid fatty substance or substances and the wax (es) being chosen so as to verify the following relationship:

$$0 \leq \Delta\delta \leq 5$$

in which:

$$\Delta\delta = (4 \times (\delta D \text{ waxes} - \delta D \text{ oils})^2 + (\delta P \text{ waxes} - \delta P \text{ oils})^2 - (\delta H \text{ waxes} - \delta H \text{ oils})^2]^4, \text{ where:}$$

- $\delta D$ characterizes the London dispersion forces resulting from the formation of dipoles induced during molecular impacts;
- $\delta P$ characterizes the Debye interaction forces between permanent dipoles;
- $\delta H$ characterizes the specific interaction forces;

the said composition comprising a particulate phase comprising from 0 to 15% by weight of pigments, from 0 to

20% by weight of pearlescent agents and from 0 to 30% by weight of fillers, with respect to the total weight of the composition.

## Patentansprüche

**1.** Topische Zusammensetzung ohne Transfer' zum Schminken der Haut, der Semischleimhäute, der Schleimhäute und/oder der Anhangsgebilde der Haut in wasserfreier Form, die eine Fettphase enthält, die 8 bis 99 Gew.-% einer flüchtigen Verbindung, mindestens ein Wachs, mindestens eine nicht flüchtige, flüssige Fettsubstanz und eine Partikelphase aufweist,

- wobei die Fettphase mindestens ein Fluorsilicon der Formel (III) enthält:

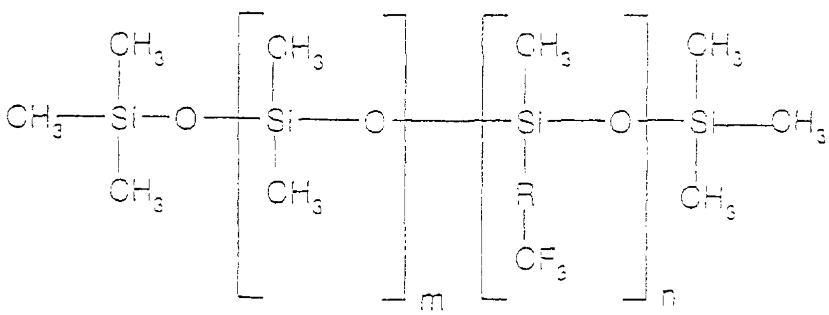

worin:

- R eine zweiwertige Methyl-, Ethyl-, Propyl- oder Butylgruppe bedeutet,
- m im Bereich von 0 bis 80 ausgewählt ist, und
- n im Bereich von 1 bis 30 ausgewählt ist.
- die nicht flüchtige(n), flüssige(n) Fettsubstanz(en) und/oder das oder die Wachs(e) so ausgewählt sind, daß sie die folgende Beziehung erfüllen:

$$0 \leq \Delta\delta \leq 5$$

worin bedeuten:

$$\Delta\delta = [(4 \text{ x } \delta D \text{ Wachse} - \delta D \text{ Öle})^2 + [(\delta P \text{ Wachse} - \delta P \text{ Öle})^2 + [(\delta H \text{ Wachse} - \delta H \text{ Öle})^2]^{1/2},$$

wobei:

- δD die London-Dispersionskräfte angibt, die aus der Bildung von Dipolen resultieren, die durch Molekülstöße induziert werden;
- δP die Debye-Wechselwirkungskräfte zwischen permanenten Dipolen kennzeichnet;
- δH spezielle Wechselwirkungskräfte angibt, und
- die Partikelphase 0 bis 15 Gew.-% Pigmente 0 bis 20 Gew.-% Perlglanzpigmente und 0 bis 30 Gew.-% Füllstoffe, bezogen auf das Gesamtgewicht der Zusammensetzung, enthält.

**2.** Zusammensetzung nach Anspruch 1, wobei das Fluorsilicon unter den flüchtigen Verbindungen, nicht flüchtigen, flüssigen Fettsubstanzen, Wachsen und deren Gemischen ausgewählt ist.

**3.** Zusammensetzung nach einem der vorhergehenden Ansprüche, wobei das Fluorsilicon in Form einer nicht flüchtigen flüssigen Fettsubstanz vorliegt.

**4.** Zusammensetzung nach einem der vorhergehenden Ansprüche, wobei die Fettphase mindestens ein fluoriertes

Kohlenwasserstoffwachs enthält.

**5.** Zusammensetzung nach einem der vorhergehenden Ansprüche, wobei das Fluorsilicon in der Zusammensetzung in Mengenanteilen von 0,1 bis 40 Gew.-% und vorzugsweise 3 bis 30 Gew.-% vorliegt.

**6.** Zusammensetzung nach einem der vorhergehenden Ansprüche, wobei die flüchtige Verbindung unter den Kohlenwasserstoffölen und/oder den cyclischen oder linearen Siliconölen oder deren Gemischen ausgewählt ist.

**7.** Zusammensetzung nach einem der vorhergehenden Ansprüche, wobei das Wachs unter den tierischen, pflanzlichen, mineralischen und synthetischen Wachsen und deren Gemischen ausgewählt ist, wie z.B. mikrokristallinen Wachsen, Paraffin, Petrolatum, Vaseline, Ozokerit, Montanwachs, Bienenwachs, Lanolin und seinen Derivaten, Candelillawachs, Ouricurywachs, Carnaubawachs, Japanwachs, Cacaobutter, Wachsen aus Korkfasem oder Zukkerrohr, bei 25 °C festen, hydrierten Ölen, Ozokeriten, Fettsäureestern und Glyceriden, die bei 25 °C fest sind, Polyethylenwachsen und durch Fischer-Tropsch-Synthese hergestellten Wachsen, Siliconwachsen und insbesondere Alkylsiliconen.

**8.** Zusammensetzung nach einem der vorhergehenden Ansprüche, wobei sie ferner mindestens ein Öl enthält, das unter den nicht flüchtigen Kohlenwasserstoffölen und/oder den nicht flüchtigen, phenylgruppenhaltigen Siliconölen ausgewählt ist.

**9.** Zusammensetzung nach einem der vorhergehenden Ansprüche, wobei die in der Zusammensetzung vorliegenden nicht flüchtigen Öle hauptsächlich Kohlenwasserstofföle sind.

**10.** Zusammensetzung nach einem der vorhergehenden Ansprüche mit $\Delta\delta \leq 4$.

**11.** Zusammensetzung nach einem der vorhergehenden Ansprüche, die als Stick oder Stift, in Form einer öligen, gegebenenfalls gelierten Flüssigkeit oder auch in Form einer weichen Paste vorliegt, deren dynamische Viskosität bei 25 °C im Bereich von 1 bis 40 Pa·s und besser noch 3 bis 35 Pa·s liegt, wobei die Viskosität mit einem Rotationsviskometer CONTRAVES TV, das mit einem beweglichen Teil MS-r4 ausgestattet ist, bei einer Frequenz von 60 Hz bestimmt wird.

**12.** Zusammensetzung nach einem der vorhergehenden Ansprüche, die als Lippenstift, Make-up, Wangenrouge, Lidschatten, Mascara, Eyeliner, Lippenpflegebasis, Fixiermasse, Pflegeprodukt, Hygieneprodukt, pharmazeutisches Produkt, Selbstbräunungsmittel oder Produkt zum Sonnenschutz vorliegt.

**13.** Zusammensetzung nach einem der vorhergehenden Ansprüche, dadurch gekennzeichnet, daß es sich um eine Zusammensetzung zum Schminken der Lippen handelt.

**14.** Verwendung, einer Fettphase, die mindestens ein Fluorsilicon, 8 bis 99 Gew.-% einer flüchtigen Verbindung, mindestens ein Wachs, mindestens eine nicht flüchtige, flüssige Fettsubstanz und eine Partikelphase aufweist, in einer in wasserfreier Form vorliegenden, topischen Zusammensetzung 'ohne Transfer' zum Schminken der Haut, der Semischleimhäute, der Schleimhäute und/oder der Anhangsgebilde der Haut, wobei die nicht flüchtige(n), flüssige(n) Fettsubstanz(en) und/oder das oder die Wachs(e) so ausgewählt sind, daß sie die folgende Beziehung gemäß der in Anspruch 1 angegebenen Definition erfüllen: $0 \leq \Delta\delta \leq 5$, das Fluorsilicon der Formel (III) entspricht:

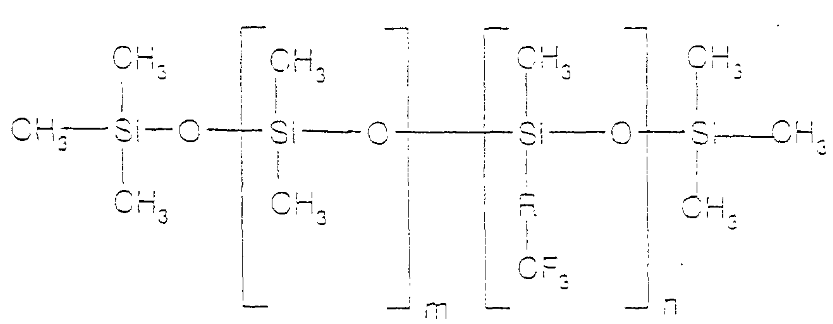

worin:

- R eine zweiwertige Methyl-, Ethyl-, Propyl- oder Butylgruppe bedeutet,
- m im Bereich von 0 bis 80 ausgewählt ist, und
- n im Bereich von 1 bis 30 ausgewählt ist, und
- die Partikelphase 0 bis 15 Gew.-% Pigmente, 0 bis 20 Gew.-% Perlglanzpigmente und 0 bis 30 Gew.-% Füllstoffe, bezogen auf das Gesamtgewicht der Zusammensetzung, enthält,

zur Herstellung einer Zusammensetzung, die gute Eigenschaften bezüglich der Glätte, des Nichtspannens, der Weichheit und des Anwendungskomforts aufweist.

**15.** Verwendung nach Anspruch 14, wobei das Fluorsilicon unter den flüchtigen Verbindungen, nicht flüchtigen flüssigen Fettsubstanzen, Wachsen und deren Gemischen ausgewählt ist.

**16.** Verwendung nach einem der Ansprüche 14 bis 15, wobei die Zusammensetzung mindestens ein fluoriertes Kohlenwasserstoffwachs enthält.

**17.** Verwendung nach einem der Ansprüche 14 bis 16, wobei das Fluorsilicon in der Zusammensetzung in Mengenanteilen von 0,1 bis 40 Gew.-% und vorzugsweise 3 bis 30 Gew.-% vorliegt.

**18.** Verwendung nach einem der Ansprüche 14 bis 17, wobei die Zusammensetzung ferner mindestens eine Öl enthält, die unter den nicht flüchtigen Kohlenwasserstoffölen und/oder den nicht flüchtigen, phenylgruppenhaltigen Siliconölen ausgewählt ist.

**19.** Verwendung nach einem der Ansprüche 14 bis 18, wobei die in der Zusammensetzung vorliegenden nicht flüchtigen Öle hauptsächlich Kohlenwasserstofföle sind.

**20.** Verwendung nach einem der Ansprüche 14 bis 19 mit $\Delta\delta \leq 4$.

**21.** Verwendung nach einem der Ansprüche 14 bis 20, wobei die Zusammensetzung als Lippenstift, Make-up, Wangenrouge, Lidschatten, Mascara, Eyeliner, Lippenpflegebasis, Fixiermasse, Pflegeprodukt, Hygieneprodukt, pharmazeutisches Produkt, Selbstbräunungsmittel oder Produkt zum Sonnenschutz vorliegt.

**22.** Nicht therapeutisches kosmetisches Verfahren, um den Transfer einer wasserfreien kosmetischen Zusammensetzung zum Schminken der Haut, der Schleimhäute, der Semischleimhäute und/oder der Anhangsgebilde der Haut zu beschränken, zu vermindern und/oder zu verhindern, das darin besteht, in die Zusammensetzung eine Fettphase einzuarbeiten, die 8 bis 99 Gew.-% einer flüchtigen Verbindung, mindestens ein Wachs, mindestens eine nicht flüchtige, flüssige Fettsubstanz und mindestens ein Fluorsilicon der Formel (III) enthält:

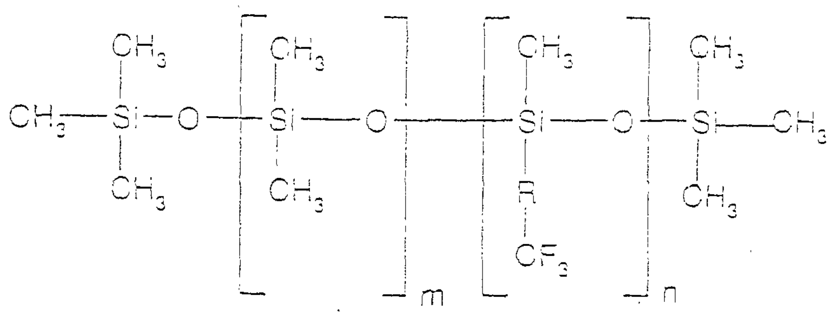

worin:

- R eine zweiwertige Methyl-, Ethyl-, Propyl- oder Butylgruppe bedeutet,
- m im Bereich von 0 bis 80 ausgewählt ist, und
- n im Bereich von 1 bis 30 ausgewählt ist;
- die nicht flüchtige(n), flüssige(n) Fettsubstanz(en) und/oder Wachs(e) so ausgewählt sind, daß sie die folgende Beziehung erfüllen:

$$0 \leq \Delta\delta \leq 5$$

worin bedeuten:

$$\Delta\delta = [(4 \text{ x } \delta D \text{ Wachse} - \delta D \text{ Öle})^2 + [(\delta P \text{ Wachse} - \delta P \text{ Öle})^2 + [(\delta H \text{ Wachse} - \delta H \text{ Öle})^2]^{1/2},$$

wobei:

- $\delta D$ die London-Dispersionskräfte angibt, die aus der Bildung von Dipolen resultieren, die durch Molekülstöße induziert werden;
- $\delta P$ die Debye-Wechselwirkungskräfte permanenten Dipolen kennzeichnet;
- $\delta H$ spezielle Wechselwirkungskräfte angibt, und
- wobei die Zusammensetzung eine Partikelphase enthält, die 0 bis 15 Gew.-% Pigmente, 0 bis 20 Gew.-% Perlglanzpigmente und 0 bis 30 Gew.-% Füllstoffe, bezogen auf das Gesamtgewicht der Zusammensetzung, enthält.